(19) Europäisches Patentamt
European Patent Office
Office européen des brevets

(11) EP 1 843 735 B1

# (12) EUROPÄISCHE PATENTSCHRIFT

(45) Veröffentlichungstag und Bekanntmachung des Hinweises auf die Patenterteilung:
**21.01.2009 Patentblatt 2009/04**

(21) Anmeldenummer: **06754077.3**

(22) Anmeldetag: **02.06.2006**

(51) Int Cl.:
***A61K 6/00*** *(2006.01)*

(86) Internationale Anmeldenummer:
**PCT/EP2006/005280**

(87) Internationale Veröffentlichungsnummer:
**WO 2006/136277 (28.12.2006 Gazette 2006/52)**

## (54) HAARPFLEGEMITTEL

HAIR CARE AGENTS

PRODUIT DE SOIN POUR CHEVEUX

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **20.06.2005 DE 102005028622**

(43) Veröffentlichungstag der Anmeldung:
**17.10.2007 Patentblatt 2007/42**

(73) Patentinhaber: **Henkel AG & Co. KGaA**
**40589 Düsseldorf (DE)**

(72) Erfinder:
- **HÖFFKES, Horst**
  **40595 Düsseldorf (DE)**
- **GOUTSIS, Konstantin**
  **41812 Erkelenz (DE)**

(56) Entgegenhaltungen:
**EP-A- 1 284 135**
**EP-A- 1 447 075**
**FR-A- 2 837 697**
**US-A- 5 006 332**
**US-B1- 6 221 389**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 843 735 B1

Printed by Jouve, 75001 PARIS (FR)

## Beschreibung

Die vorliegende Erfindung betrifft Mittel zur Konditionierung keratinhaltiger Fasern, die ein spezielles amphoteres Tensid in Kombination mit mindestens einem amphoteren Polymer enthalten, eine Verpackungseinheit (Kit) enthaltend das erfindungsgemäße Mittel sowie ein Verfahren zur Haarbehandlung mit diesem Mittel.

Nicht zuletzt durch die starke Beanspruchung der Haare, beispielsweise durch Umweltbelastungen, das Färben oder Dauerwellen als auch durch die Reinigung der Haare mit Shampoos, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Behandlung zu unterziehen. Dabei werden, üblicherweise in Form einer Spülung, die Haare mit speziellen Pflegestoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung oder Färbung der Haare, zusätzlich Pflegestoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Pflegestoffe sowohl für separate Nachbehandlungsmittel als auch für Kombinationspräparate wirken im Allgemeinen bevorzugt an der Haaroberfläche. So sind Pflegestoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann.

Es besteht daher weiterhin ein Bedarf nach Pflegestoffen bzw. Pflegestoffkombinationen für kosmetische Mittel mit guten pflegenden Eigenschaften und guter biologischer Abbaubarkeit.

Pflegestoffe können in die verschiedensten kosmetischen Träger eingearbeitet werden. Als kosmetische Träger dienen dabei Lotionen, Shampoos oder O/W- bzw. W/O-Emulsionen. Insbesondere die Form eines klaren Gels stellt einen speziellen, reizvollen kosmetischen Träger für Pflegestoffe dar.

Wenn Konditioniermittel in Form eines klaren Gels formuliert werden sollen, stellt sich für den Fachmann die Aufgabe, die Pflegestoffe des Konditioniermittels so auszuwählen, dass sich der Pflegestoff dauerhaft in den gelförmigen kosmetischen Träger einarbeiten lässt. Das resultierende Gel sollte stets stabil und klar bleiben, d.h. z.B. bei längerer Lagerung, insbesondere bei Temperaturen von -20°C bis 60°C, sollte keine Phasentrennung oder Trübung auftreten. Die Pflegeleistung darf bei Berücksichtigung der vorherigen Gesichtspunkte nicht beeinträchtigt werden.

Die Konditioniermittel sollen sich ebenso hervorragend zur Formulierung von Kombinationspräparaten, insbesondere Kombinationspräparate auf Basis oxidativer Färbemittel, eignen.

Für das Färben von Keratinfasern, insbesondere von menschlichen Haaren, spielen die so genannten Oxidationsfärbemittel wegen ihrer intensiven Farben und guten Echtheitseigenschaften eine bevorzugte Rolle. Solche Färbemittel enthalten Oxidationsfarbstoffvorprodukte, so genannte Entwicklerkomponenten und Kupplerkomponenten.

Die Entwicklerkomponenten tragen, wenn sie als Aromaten vorliegen, mindestens eine gegebenenfalls substituierte Aminogruppe und mindestens eine dazu in ortho- oder para-Position befindliche Amino- oder Hydroxygruppe, welche gegebenenfalls ebenso substituiert sein kann.

Spezielle Vertreter der Entwicklerkomponenten sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diamino-propan-2-ol.

Als Kupplerkomponenten werden in der Regel m-Phenylendiaminderivate, Naphthole, Resorcin und Resorcinderivate, Pyridine, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, 2-Amino-3-hydroxypyridin, m-Phenylendiamin, 1-Phenyl-3-methylpyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol. Die Kupplerkomponenten tragen, wenn sie als aromatische Verbindungen vorliegen, keine zueinander ortho- oder paraständigen Amino- bzw. Hydroxygruppen, welche gegebenenfalls substituiert sein können.

Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Meist wird

ein Oxidationsmittel wie Wasserstoffperoxid direkt vor der Anwendung den Oxidationsfarbstoffvorprodukten zugemischt.

Die oxidativen Färbemittel werden daher oft als Verkaufseinheit (Kit) mit mindestens zwei getrennt konfektionierten Komponenten angeboten:

Die Oxidationsfarbstoffvorprodukte sind als erste Komponente in einer meist auf einen basischen pH-Wert eingestellten Färbecreme oder einem Färbegel enthalten. Färbegele sind nicht nur ästhetischer und daher ansprechender für den Verbraucher. Zusätzlich bieten Gele aufgrund ihrer Strukturviskosität einen Vorteil bei der Anwendung am Haar. Besonders wichtig ist daher, dass nach dem Auftragen auf die Fasern die Strukturviskosität des erfindungsgemäßen Konditioniermittels erhalten bleibt und letzteres sich zwar leicht auf dem Haar verteilen läßt, nicht aber vom Haar abläuft.

Die Oxidationsmittel sind in diesem Falle in einer gesondert verpackten Oxidationsmittelzubereitung, meist im Form einer Lotion, Emulsion oder Dispersion konfektioniert, als zweite Komponente in dem Kit enthalten. Die Oxidationsmittelzubereitung besitzt üblicherweise einen sauren pH-Wert.

Die Anwendungsmischung aus der ersten und zweiten Komponente besitzt üblicherweise einen basischen pH-Wert. Die Pflegestoffe müssen folglich auch bei basischem pH-Wert eine angemessene Pflegeleistung erbringen. Erstrebenswerterweise liefern die Pflegestoffe auch in Gegenwart von Oxidationsmitteln, wie z.B. Wasserstoffperoxid, und/oder hohen Elektrolytkonzentrationen ein hervorragendes Pflegeergebnis.

Gute oxidative Färbemittel bilden bei der oxidativen Kupplung der Vorprodukte die gewünschten Farbnuancen in ausreichender Intensität und Echtheit aus. Die Oxidationsfarbstoffvorprodukte sollen ferner ein gutes Aufziehvermögen auf die Faser besitzen, wobei insbesondere bei menschlichen Haaren keine merklichen Unterschiede zwischen strapaziertem und frisch nachgewachsenem Haar bestehen sollen (Egalisiervermögen). Die erzielten Färbungen sollen beständig sein gegen Licht, Wärme, Schweiß, Reibung und den Einfluss chemischer Reduktionsmittel, z.B. Dauerwellenflüssigkeiten. Schließlich sollen die Mittel - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Zusätzlich zu hervorragenden färberischen Eigenschaften stellt der Verbraucher an die modernen oxidativen Färbemittel den Anspruch der Haarpflege.

Wie in Färbeverfahren mit herkömmlichen oxidativen Haarfärbemitteln üblich, wird nach dem Färbevorgang ein sogenanntes Konditioniermittel im Rahmen einer Nachbehandlung angewendet. Diese Konditioniermittel weisen einen sauren bis neutralen pH-Wert auf und sind oft in kommerziell angebotenen oxidativen Färbemittel-Kits enthalten. Diese Konditioniermittel dienen der Avivage und sollen in manchen Fällen den Farberhalt verbessern.

Seit geraumer Zeit sind oxidative Haarfärbemittel bekannt, welche neben der Haarfärbung gleichzeitig avivierend wirken. Die Haarpflege (Avivage) wird bei diesen 2-in-1-Färbemitteln meist durch Zusatz von bekannten Pflegestoffen zum oxidativen Haarfärbemittel erreicht. Im handelsüblichen Haarfärbe-Kit werden die Pflegestoffe entweder der oxidationsmittelhaltigen Komponente, separat konfektioniert bzw. der Färbecreme zugesetzt, je nach dem, ob der betroffene Pflegestoff lagerstabil in eine Oxidationsmittelzubereitung eingearbeitet werden kann oder nicht.

Oft erfolgt auch bei Anwendung der 2-in-1-Färbemittel nach der oxidativen Färbung eine Nachbehandlung mit einem sauer eingestellten Konditioniermittel.

Ein Problem der 2-in-1-Färbemittel ist es, dass die darin enthaltenen Pflegestoffe die Farbaufnahme des Haars beeinträchtigen können. Dies kann insbesondere bei polymeren Pflegestoffen der Fall sein. Darüber hinaus können möglicherweise die zugesetzten Pflegestoffe die Eigenschaften der Färbungen negativ beeinflussen.

Die Druckschrift US-A-4 507 280 betrifft Haarkonditioniermittel, welche neben 0.1 bis 10 % eines kationischen Polymers, 0.2 bis 20 % eines amphoteren Tensids in einem definierten Mengenverhältnis zueinander enthalten. Die Konditioniermittel besitzen einen sauren pH-Wert. Ferner sind die Pflegestoffe nicht in einen klaren förmigen Träger eingearbeitet. Die Druckschrift US 6221 1389 betrifft Heermittel welche MIRANOL C2M-SF (disodium cocoamphodiproprionate) enthalten.

Aus der Druckschrift EP-B1-640 335 sind Zweikomponenten-Färbemittel bekannt, welche in einer ersten Komponente eine alkalische wässrige Lösung von Entwickler und Kupplerkomponenten in Kombination mit einem kationischen Polymer, einem anionischen und/oder amphoteren Tensid und mindestens 70% Wasser enthalten. In einer zweiten, auf einen sauren pH-Wert eingestellten Komponente sind mindestens ein Oxidationsmittel und ein dispergiertes anionisches Polymer enthalten. Nach der Mischung der Komponenten bildet sich ein Gel mit einem pH-Wert von größer pH 7. Dabei löst sich das anionische Polymer auf, das kationische Polymer jedoch bildet einen unlöslichen Niederschlag.

Aufgabe der vorliegenden Erfindung ist es, neue (insbesondere gelförmige und klare) Konditioniermittel als pflegend wirkende Oxidationsfärbemittel für keratinhaltige Fasern bereitzustellen, die lagerstabil sind und zusätzlich hervorragende haarkonditionierende Eigenschaften, insbesondere bei einer oder mehrerer der eingangs beschriebenen Bedingungen, besitzen. Die Konditioniermittel sollen zusätzlich Färbungen mit guten Echtheitseigenschaften, insbesondere in Bezug auf die Wasch-, Reib-, Licht-, Kaltwell- und Schweißechtheit, in einem breiten Farbspektrum liefern und in dermatologischer Hinsicht unproblematisch sein.

Ein erster Gegenstand der Erfindung sind wasserhaltige Mittel zur Konditionierung keratinhaltiger Fasern, insbesondere menschlicher Haare, enthaltend eine Kombination aus

- mindestens einem amphoteren Tensid gemäß Formel **(I)** und/oder dessen physiologisch verträglichem Salz,

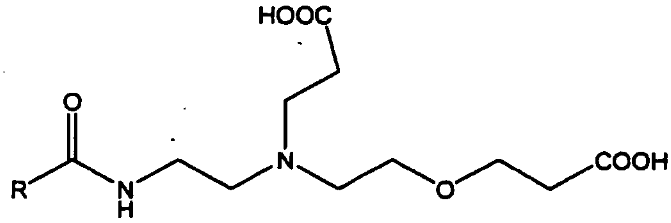

**(I)**

worin R eine lineare oder verzweigte, gesättigte oder ungesättigte ($C_{10}$ - $C_{22}$)-Alkylgruppe bedeutet,
- mindestens einem amphoteren Polymer.
und
- mindestens eine Entwickleskomponente als Oxidationsfarbstoffvorprodukt und gegebenfalls mindestens eine Kupplerkomponente.

Das erfindungsgemäße Mittel bewirkt eine hervorragende Avivage, insbesondere eine verbesserte Nasskämmbarkeit. Wenn die Konditioniermittel als Gel vorliegen, wird ein klares und lagerstabiles Gel erhalten.

Unter keratinhaltigen Fasern sind erfindungsgemäß Pelze, Wolle, Federn und insbesondere menschliche Haare zu verstehen. Obwohl sich die erfindungsgemäßen Konditioniermittel in erster Linie zur Konditionierung von keratinhaltigen Fasern eignen, steht prinzipiell einer Verwendung auch auf anderen Gebieten nichts entgegen.

Ein erfindungsgemäßes wasserhaltiges Mittel enthält bevorzugt Wasser in einer Menge von 50 bis 99 Gew.-%, besonders bevorzugt von 60 bis 85 Gew.-%, jeweils bezogen auf das Gewicht des Mittels.

Die Verbindungen der Formel **(I)** sind in dem erfindungsgemäßen Mittel bevorzugt in einer Menge von 0,05 bis 30 Gew.-%, besonders bevorzugt von 0,1 bis 20 Gew.-%, jeweils bezogen auf das Gewicht des Mittels, enthalten.

Die amphoteren Polymere sind in dem erfindungsgemäßen Mittel bevorzugt in einer Menge von 0,01 bis

Das Mengenverhältnis der Verbindung mit der Formel (I) zum amphoteren Polymer beträgt bevorzugt 1 zu 5 bis 5 zu 1, besonders bevorzugt 1 zu 1 bis 1 zu 2.

In einer bevorzugten Ausführungsform steht der Rest R gemäß Formel (I) für eine lineare ($C_{12}$ bis $C_{18}$)-Alkylgruppe.

Besonders bevorzugte Verbindungen der Formel (I) werden unter der INCI-Bezeichnung Disodium Cocoamphodipropionate mit den Handelsnamen Miranol® C2M SF conc. (Rhodia), Amphoterge® K-2 (Lonza) und Monateric® CEM-38 (Unichema) vermarktet. Dabei leitet sich die Gruppe R-CO- gemäß Formel (I) von Fettsäuren des Kokosnußöls ab.

Unter dem Begriff amphotere Polymere werden solche Polymere verstanden,
- die im Molekül sowohl freie Aminogruppen als auch freie -COOH- oder $SO_3H$-Gruppen enthalten und zur Ausbildung innerer Salze befähigt sind,
- zwitterionische Polymere, die im Molekül quartäre Ammoniumgruppen und $-COO^-$-Gruppen oder $-SO3^-$-Gruppen enthalten, sowie
- Polymere, die -COOH-Gruppen oder $SO_3H$-Gruppen und quartäre Ammoniumgruppen enthalten.

Die oben genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere Polymere sind solche Polymerisate, in denen sich eine kationische Gruppe ableitet von mindestens einem der folgenden Monomere:

(M1) Monomeren mit quartären Ammoniumgruppen der allgemeinen Formel (II),

$$R^1\text{-}CH{=}CR^2\text{-}CO\text{-}Z\text{-}(C_nH_{2n})\text{-}N^{(+)}R^3R^4R^5\ A^{(-)} \quad \text{(II)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,

(M2) Monomeren mit quartären Ammoniumgruppen der allgemeinen Formel (III),

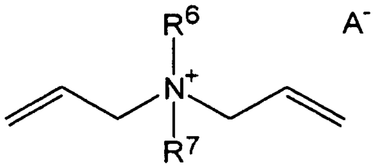

(III)

worin $R^6$ und $R^7$ unabhängig voneinander stehen für eine ($C_1$ bis $C_4$)-Alkylgruppe, insbesondere für eine Methylgruppe und
$A^-$ das Anion einer organischen oder anorganischen Säure ist.
Wenn die kationische Gruppe der amphoteren Polymerisate sich vom Monomer des Typs (M1) ableiten, stehen in Formel (II) die Reste $R^3$, $R^4$ und $R^5$ bevorzugt für Methylgruppen, Z ist bevorzugt eine NH-Gruppe und $A^{(-)}$ bedeutet bevorzugt ein Halogenid-, Methoxysulfat- oder Ethoxysulfat-Ion. Besonders bevorzugt ist es in diesem Falle Acrylamidopropyl-trimethyl-ammoniumchlorid als Monomer (M1) zu verwenden.
In Formel (III) steht $A^-$ bevorzugt für ein Halogenidion, insbesondere für Chlorid oder Bromid.
Bevorzugte erfindungsgemäße amphotere Polymere sind Polymere, deren anionische Gruppe sich von mindestens einem Monomeren der Formel (IV) ableitet

(M3) monomeren Carbonsäuren der allgemeinen Formel (IV) bzw. deren Salze mit einer organischen oder anorganischen Säure,

$$R^8\text{-}CH{=}CR^8\text{-}COOH \quad \text{(IV)}$$

in denen $R^8$ und $R^9$ unabhängig voneinander Wasserstoff oder Methylgruppen sind.

Als Monomeres (M3) wird für die erfindungsgemäß bevorzugten amphoteren Polymerisate Acrylsäure verwendet.

Besonders bevorzugte Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Die amphoteren Polymere können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

In einer bevorzugten Ausführungsform der Erfindung liegt das erfindungsgemäße Konditioniermittel als klares Gel vor.

Der Begriff "Gel" wird in "Faraday discussions of the chemical society, No. 57, 1974 (Gels and Gelling Processes), P.J. Flory, Seite 7-18", näher charakterisiert. Demnach werden Gele in 4 Untergruppen eingeteilt:

1. Gele mit lamellaren Ordnungsstrukturen einschließlich Gel-Mesophasen, z.B. Tensidgele, insbesondere Seifengele.
2. Gele, die aus polymeren, dreidimensionalen, kovalent gebundenen Netzwerken, die ohne Zerstörung der Kovalenzbindungen in Lösungsmitteln unlöslich sind, aufgebaut sind.
3. Gele aus polymeren Netzwerken mit lokalen Ordnungsstrukturen, die durch physikalische Kräfte zusammengehalten werden, z.B. Gelatine, Homo- und Copolymere von Acryl- und Methacrylsäure, Alginate, Carrageenan.
4. Gele gebildet aus feinverteilten Bestandteilen, z.B. Niederschlägen, die i.a. eine große geometrische Anisotropie aufweisen, z.B. $V_2O_5$-Gele oder Gele, die sich durch Aggregation von Proteinen bilden.

Gele im Sinne dieser Ausführungsform der Erfindung sind solche, die unter Punkt 1. und Punkt 3. der obigen Auflistung fallen. Als "Gel" im Sinne der vorliegenden Erfindung sind unter anderem Transparentgele wie sie z.B. in den US-Patentschriften US 3 101 300 und US 3 101 301 beschrieben werden, zu verstehen. Diese Transparentgele werden als Mikroemulsionsgele bezeichnet. Eine allgemeingültige Definition für Mikroemufsionsgele ist in "Happi: Household Pers. Prod. Ind. 30 (1993), Nr. 2, p. 58-64" angegeben.

Ein Gel ist erfindungsgemäß klar, wenn bei einer Schichtdicke von 1 cm die Extinktion bei einer Wellenlänge, in dem die gelförmige Zusammensetzung keine Absorptionsbanden aufweist (typischerweise bei einer Wellenlänge von 800 nm) höchstens 0.05 Extinktionseinheiten beträgt.

Das Gel sollte vorzugsweise fließfähig sein, und durch eine hohe Strukturviskosität gekennzeichnet sein, d.h., diese Zubereitungen erscheinen unter dem Einfluß geringer Scherkräfte, etwa des Eigengewichts, relativ hochviskos, lassen sich aber bei Anwendung höherer Scherkräfte, z.B. beim Verrühren sehr leicht bewegen.

Es ist erfindungsgemäß bevorzugt, als klare, wässrige Gelgrundlage eine Zusammensetzung, enthaltend

a) 0,5 bis 10 Gew.-% eines Anlagerungsproduktes von 1 bis 5 Mol Ethylenoxid an einen linearen Fettalkohol mit 8 bis 22 C-Atomen,

b) 6,0 bis 15 Gew.-% eines gesättigten oder ungesättigten, linearen oder verzweigten Alkohols mit 8 bis 36 C-Atomen und

c) 0,1 bis 15 Gew.-% einer flüssigen Fettsäure mit 16 bis 22 C-Atomen in Form einer wasserlöslichen Seife

zu verwenden. Dieses Transparentgel eignet sich besonders gut zur Einarbeitung der erfindungsgemäßen Kombination aus amphoterem Tensid und amphoterem Polymer.

Erfindungsgemäß verwendbare Anlagerungsprodukte von 1 bis 5 Mol Ethylenoxid an einen linearen Fettalkohol mit 8 bis 22 C-Atomen werden beispielsweise mit der INCI-Bezeichnung Laureth-2 (Handelsprodukt Dehydol® LS 2 der Fa. Cognis), Laureth-3 (Handelsprodukt Dehydol® LS 3 der Fa. Cognis), Laureth-4 (Handelsprodukt Dehydol® LS 4 der Fa. Cognis), Ceteareth-2 (Lowenol® C 279 der Fa. Lowenstein), Steareth-2 (Eumulgin® S 2 der Fa. Cognis) bzw. Steareth-4 (Arlypon® SA 4 der Fa. Cognis) bekannt.

Bei den erfindungsgemäß einzusetzenden gesättigten oder ungesättigten, linearen oder verzweigten Alkoholen mit 8 bis 36 C-Atomen handelt es sich vorzugsweise um Fettalkohole und/ oder Guerbetalkohole.

Unter Fettalkoholen sind primäre aliphatische Alkohole der Formel (V) zu verstehen,

$$R^{10}OH \qquad (V)$$

in der $R^{10}$ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkoholmischungen mit 12 bis 18 Kohlenstoffatomen wie beispielsweise Kokos-, Palm-, Palmkern- oder Talgfettalkohol, insbesondere Kokos- und/ oder Talgfettalkohol.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Die zur Seifenbildung geeigneten Fettsäuren sind bevorzugt flüssige oder niedrigschmelzende ungesättigte lineare $C_{16}$-$C_{22}$-Fettsäuren wie Palmitoleinsäure, Ölsäure, Elaidinsäure, Myristinsäure, Petroselinsäure, Petroselaidinsäure, Gadoleinsäure, Erucasäure, Brassidinsäure sowie Gemische dieser Fettsäuren untereinander und gegebenenfalls mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyldecansäure, Isostearinsäure und die 2-Octyldodecansäure.

Zur Überführung der Fettsäuren in wasserlösliche Seifen eignen sich Alkalihydroxide und Alkalicarbonate, Ammoniak, Mono-, Di- und Trialkanolamine mit 2 bis 4 C-Atomen in der Alkanolgruppe sowie alkalische Aminosäuren

wie beispielsweise Arginin, Ornithin, Lysin und/ oder Histidin.

Es ist erfindungsgemäß bevorzugt, wenn das Konditioniermittel zusätzlich, wiederum bevorzugt zusammen mit den drei vorgenannten Komponenten a) bis c), 0,5 bis 10 Gew.-% eines Anlagerungsproduktes von 15 bis 100 Einheiten Ethylenoxid, insbesondere von 15 bis 50 Mol Ethylenoxid jeweils pro Molekül eines linearen Fettalkohols mit 8 bis 22 C-Atomen, in einer wasserhaltigen Zusammensetzung enthält. Es handelt sich dabei ganz besonders bevorzugt um Ceteareth-15 oder Ceteareth-50, welche als Eumulgin® CS 15 bzw. Eumulgin® CS 50 von der Firma Cognis Deutschland GmbH vermarktet werden.

Als Anlagerungsprodukte von 1 bis 4 bzw. 15 bis 100 Mol Ethylenoxid an einen linearen Fettalkohol mit 12 bis 22 C-Atomen eignen sich alle nach den bekannten technischen Oxethylierungsverfahren erhältlichen Addukte. Bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Die erfindungsgemäßen Mittel sind wasserhaltige Zusammensetzungen. Darunter fallen auch wässrig-alkoholische Systeme. Unter wässrig-alkoholisch sind im Sinne der vorliegenden Erfindung wasserhaltige Zusammensetzungen enthaltend 3 bis 70 Gew.-% eines niedermolekularen Monoalkohols und/oder eines Polyols zu verstehen.

Unter niedermolekularen Monoalkoholen sind im Sinne der vorliegenden Erfindung mit Wasser mischbare Alkohole mit 1 bis 5 C-Atomen zu verstehen. Diese Monoalkohole tragen nur eine Hydroxygruppe. Vorzugsweise handelt es sich bei den niedermolekularen Monoalkoholen um Ethanol, Propanol und/ oder Isopropanol.

Polyole tragen im Sinne der Erfindung mindestens zwei Hydroxygruppen, bevorzugt zwei bis vier Hydroxygruppen. Als Polyole sind insbesondere solche mit 2 bis 6 C-Atomen bevorzugt. Es eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist besonders bevorzugt.

Bevorzugt enthalten die erfindungsgemäßen Mittel mindestens einen niedermolekularen Monoalkohol und mindestens ein Polyol.

Als wasserlösliche synthetische Tenside eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziiert ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und $\alpha$-Olefinsulfonate mit 12 bis 18 C-Atomen. Geeignete nichtionogene Tenside sind z.B. die Anlagerungsprodukte von 7 bis 30 Mol Ethylenoxid an lineare Fettalkohole mit 12 bis 18 C-Atomen, an Fettsäuren mit 12 bis 18 C-Atomen sowie an Fettsäuremonoglyceride und an Fettsäure-sorbitanmonoester. Bevorzugt geeignete nichtionogene Tenside sind die Fettalkylaminoxide und insbesondere die Fettalkylglykoside, vorzugsweise Fettalkylglucoside. Die Fettalkylgruppe kann in den genannten Produkten 12 bis 18 C-Atome aufweisen. Besonders bevorzugt im Sinne der vorliegenden Erfindung sind auch amphotere Tenside wie beispielsweise N-Fettalkyldimethyl-glycin oder N-Fettalkylaminopropionsäure und/ oder zwitterionische Tenside, z.B. N-Fettalkyl-dimethylammoniumglycinat oder N-Fettacylaminopropyldimethylglycinat. Diese amphoteren Tenside können zusätzlich zu den Verbindungen gemäß Formel (I) in den erfindungsgemäßen Mitteln enthalten sein und sind von den Verbindungen der Formel (I) verschieden. Weiterhin bevorzugt werden Kationtenside wie quartäre Ammoniumverbindungen (QAV) insbesondere quaternierte Trialkylammoniumverbindungen mit Alkylresten einer Kettenlänge von $C_8$ bis $C_{22}$.

Es hat sich gezeigt, daß durch den Zusatz weiterer, begrenzt wasserlöslicher, nichtionogener Tenside eine Verdickung der erfindungsgemäßen Mittel, insbesondere der Gele, erreicht wird.

Zusätzlich können die erfindungsgemäßen Mittel Anlagerungsprodukte von 1 bis 4 Mol Ethylenoxid an ein Fettalkylamin enthalten. Als Anlagerungsprodukte von 1 bis 4 Mol Ethylenoxid an ein lineares Fettalkylamin mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein $C_{12}$-$C_{18}$-Kokosalkylamin.

Geeignete Verbindungen der Formel (VI)

$$R^{11}\text{-}(OC_2H_4)_x\text{-}A\text{-}(C_2H_4O)_y\text{-}R^{12} \qquad \text{(VI)}$$

in welcher A ein Sauerstoffatom ist, sind Dialkylether mit 12 bis 18 C-Atomen in den Alkylgruppen $R^{11}$ und $R^{12}$. Solche Produkte sind literaturbekannt. Noch besser geeignet sind die Produkte der Formel (VI), in welchen x oder y oder beide = 1 sind. Solche Dialkyloxethylether lassen sich durch literaturbekannte Veretherungsverfahren aus Fettalkoholen und Fettalkyloxyethanolen herstellen. Die Produkte der Formel (VI), in welchen A eine Gruppe

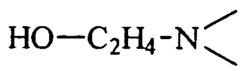

ist, lassen sich z.B. aus Triethanolamin durch O-Alkylierung mit 2 Mol eines Schwefelsäurehalbestersalzes eines ($C_{12}$ bis $C_{22}$)-Fettalkohols nach dem in DE 35 04 242 beschriebenen Verfahren zur Herstellung von Etheraminen gewinnen.

Besonders bevorzugte Verbindungen der Formel (VI) sind z.B. Dicetylstearylether, Dicetyl-stearyldioxyethylether und N,N-Bis-(2-cetyl-/stearyl-oxyethyl)-aminoethanol.

Während mit Anlagerungsprodukten von 1 bis 5 Mol Ethylenoxid an einen linearen Fettalkohol die erforderliche Verdickung meist allein durch diese Komponente erreicht wird, kann es bei Einsatz der Anlagerungsprodukte von 1 bis 4 Mol Ethylenoxid an ein lineares Fettalkylamin vorteilhaft sein, diese in Kombination mit 1 bis 10 Gew.-% einer Verbindung der Formel (VI) zu verwenden.

Desweiteren enthält das erfindungsgemäße Mittel bevorzugt zusätzlich mindestens ein anionisches Acrylsäure- und/oder Methacrylsäure-Polymerisat oder -Copolymerisat. Bevorzugte Polymerisate dieser Art sind:

- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren, wie sie z.B. in GB 870 994 beschrieben sind.
- Mischpolymerisate (aus DE 11 64 095) aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll® D (BASF).
- die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Diese anionischen Polymerisate fallen erfindungsgemäß nicht unter die Definition der erfindungsgemäßen amphoteren Polymere.

Die anionischen Acrylsäure- und/oder Methacrylsäure-Polymerisate oder - Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten.

Insbesondere bevorzugt sind in den erfindungsgemäßen Mitteln zusätzlich Proteine und/ oder Proteinderivate pflanzlicher oder tierischer Herkunft wie beispielsweise Erbsen-, Soja-, Weizen- und Mandelproteinhydrolysat oder Akazienprotein sowie Collagen- oder Keratinhydrolysat enthalten. Die erfindungsgemäßen Mittel enthalten die Proteine und/oder Proteinderivate pflanzlicher oder tierischer Herkunft bevorzugt in einer Menge von 0,1 bis 10 Gew.-%, insbesondere 0,5 bis 3 Gew.-%, jeweils bezogen auf das Gewicht des gesamten Mittels.

Weiterhin ist es bevorzugt, in die erfindungsgemäßen Mittel mindestens ein pflanzliches Öl und/oder mindestens ein Pflanzenextrakt einzuarbeiten.

Die pflanzlichen Öle werden bevorzugt durch Pressen erhalten. Bevorzugt verwendbare pflanzliche Öle sind Macadamianußöl, Kukuinußöl, Palmöl, Amaranthsamenöl, Pfirsichkernöl, Avocadoöl, Olivenöl, Kokosöl, Rapsöl, Sesamöl, Jojobaöl, Sojaöl, Erdnußöl, Nachtkerzenöl, Teebaumöl. Ein besonders bevorzugtes pflanzliches Öl ist Macadamianußöl, insbesondere das Macadamia Ternifolia Seed Oil.

Das pflanzliche Öl ist in dem erfindungsgemäßen Mittel bevorzugt in einer Menge von 0,05 bis 5 Gew.-%, insbesondere 0,1 bis 2 Gew.-%, jeweils bezogen auf das Gewicht des gesamten Mittels, enthalten.

Bevorzugt werden die Pflanzenextrakte durch Extraktion mit organischen Lösemitteln (wie beispielsweise Ethanol, Isopropanol, Diethylether, Benzin, Benzol, Chloroform) oder durch Wasserdampfdestillation gewonnen. Bevorzugte Pflanzenextrakte sind beispielsweise Extrakte von Blüten (Lindenblüten, Kamillen, Lilie, Lavendel, Rosen, Jasmin, Neroli, Ylang-Ylang), Stengeln und Blättern (Cassis, Rosskastanie, Rooibos, Birke, Melisse, Klee, Weinblättern, Geranium, Patchouli, Petitgrain), Früchten (Anis, Cassis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian, Rosmarin), Nadeln und Zweigen (Fichte, Tanne, Kiefer, Latschen, Sandelholz), Harzen und Balsamen (Galbanum, Elemi, Benzoe, Myrrhe, Olibanum, Opoponax).

Besonders bevorzugt werden die Pflanzenextrakte ausgewählt aus mindestens einem Extrakt aus der Gruppe Hamamelis (Hamamelis virginiana L.), Weinblättern (Vitis vinifera L.), Rosen (Rosa gallica L.), Sandelholz (Pterocarpus Santalinus), Rooibos (Aspalathus linearis), Rosskastanie (Aesculus Hippocastanum L.), Klee (insbesondere Rotklee, Trifolium pratense), Zimt (Cinnamomum zeylanicum nees) und Cassis (insbesondere aus Cassis Blättern, Ribes nigrum L.).

Solche bevorzugt verwendeten Extrakte werden unter den Bezeichnungen Herbasol® von der Firma Cosmetochem oder Extrapon® von der Firma Symrise vermarktet.

Die Pflanzenextrakte sind in dem erfindungsgemäßen Mittel bevorzugt in einer Menge von 0,05 bis 5 Gew.-%, insbesondere 0,1 bis 2 Gew.-%, jeweils bezogen auf das Gewicht des gesamten Mittels, enthalten.

Weiterhin kann das erfindungsgemäße Mittel zusätzlich mindestens ein Saccharid als Mono-, Di-, Oligo- und/oder Polysaccharid enthalten. Bevorzugt sind hierbei Mono-, Di- und/oder Oligosaccharide, wie insbesondere D-Glucose, D-Galactose, D-Mannose, D-Fructose, L-Arabinose, D-Xylose, D-Ribose u. 2-Desoxy-D-ribose, Saccharose, Maltose, Lactose, Galactose, Trehalose, Cellobiose, Gentiobiose und Isomaltose.

Besonders bevorzugt ist eine Kombination aus D-Glucose und D-Fructose. D-Glucose und D-Fructose können dem erfindungsgemäßen Mittel jeweils in ihrer isolierten Form oder z.B. als Honig bzw. Honigextrakt zugesetzt werden.

Das erfindungsgemäße Mittel kann zusätzlich mindestens einen direktziehenden Farbstoff, enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Amino-pyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (ent1)

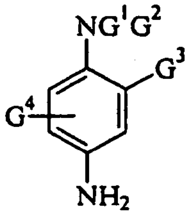

**(Ent1)**

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$-bis $C_4$-Carbamoylaminoalkoxyrest;
- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Beispiele für die als Substituenten in den erfindungsgemäßen Verbindungen genannten $C_1$- bis $C_4$-Alkylreste sind die Gruppen Methyl, Ethyl, Propyl, Isopropyl und Butyl. Ethyl und Methyl sind bevorzugte Alkylreste. Erfindungsgemäß bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, $C_1$-bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N, N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N, N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Mitteln gemäß der Erfindung verwendet werden können, kann man insbesondere die Verbindungen nennen, die der folgenden Formel (Ent2) entsprechen, sowie ihre physiologisch verträglichen Salze:

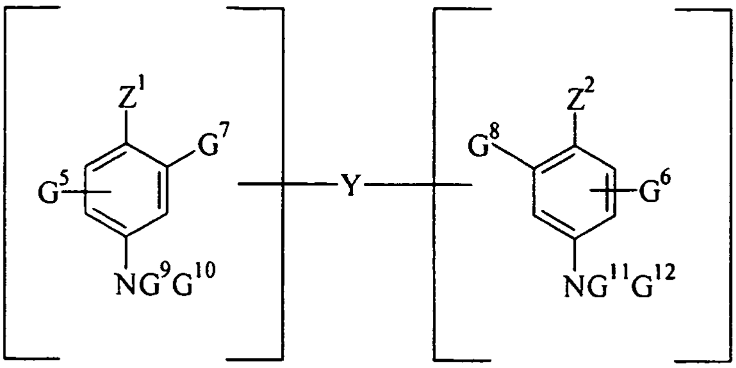

(Ent2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,
  mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3)

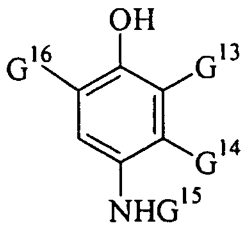

(Ent3)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$- bis $C_4$-Hydroxyalkoxyrest, einen $C_1$-bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$-bis $C_4$)-Alkoxy-($C_1$- bis $C_4$)-alkylrest, einen $C_1$- bis $C_4$-Aminoalkylrest oder einen $C_1$-bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (Ent3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-l-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl) amino-1-methylpyrazol.

Bevorzugte Pyrazolopyrimidin-Derivate sind insbesondere die Derivate des Pyrazolo[1,5-a]opyrimidin der folgenden Formel (Ent4) und dessen tautomeren Formen, sofern ein tautomeres Gleichgewicht besteht:

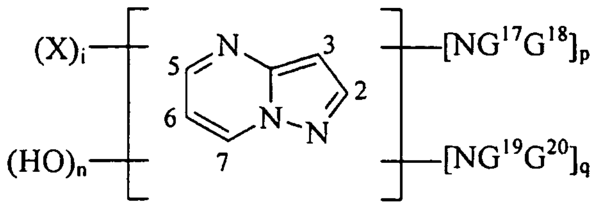

**(Ent4)**

wobei:

- $G^{17}$, $G^{18}$, $G^{19}$ und $G^{20}$ unabhängig voneinander stehen für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest einen ($C_1$- bis $C_4$)-Alkoxy-($C_1$- bis $C_4$)-alkylrest, einen $C_1$- bis $C_4$-Aminoalkylrest, der gegebenenfalls durch ein Acetyl-Ureid- oder einen Sulfonyl-Rest geschützt sein kann, einen ($C_1$- bis $C_4$)-Alkylamino-($C_1$- bis $C_4$)-alkylrest, einen Di-[($C_1$- bis $C_4$)-alkyl]-($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$)-[Hydroxyalkyl]-($C_1$- bis $C_4$)-aminoalkylrest,
- die X-Reste stehen unabhängig voneinander für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$- bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$-bis $C_4$-hydroxyalkyl)aminoalkylrest, einen Aminorest, einen $C_1$- bis $C_4$-Alkyl- oder Di-($C_1$- bis $C_4$-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
  mit der Maßgabe, dass
- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (Ent4) verwendeten Substituenten sind erfindungsgemäß analog zu den obigen Ausführungen definiert.

Wenn das Pyrazolo[1,5-a]pyrimidin der obenstehenden Formel (Ent4) eine Hydroxygruppe an einer der Positionen 2, 5 oder 7 des Ringsystems enthält, besteht ein tautomeres Gleichgewicht, das zum Beispiel im folgenden Schema dargestellt wird:

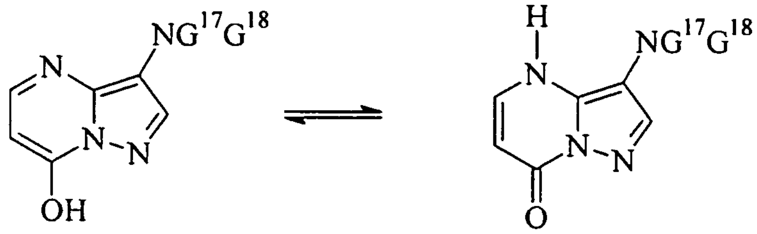

Unter den Pyrazolo[1,5-a]pyrimidinen der obenstehenden Formel (Ent4) kann man insbesondere nennen:

- Pyrazolo[1,5-a]pyrimidin-3,7-diamin;
- 2,5-Dimethyl-pyrazolo[1,5-a]pyrimidin-3,7-diamin;
- Pyrazolo[1,5-a]opyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-y1)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;

sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (Ent4) können wie in der Literatur beschrieben durch Zyklisierung ausgehend von einem Aminopyrazol oder von Hydrazin hergestellt werden.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (VIIa),

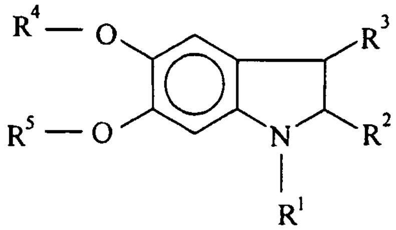

(VIIa)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^1$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und

- $R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VIIb),

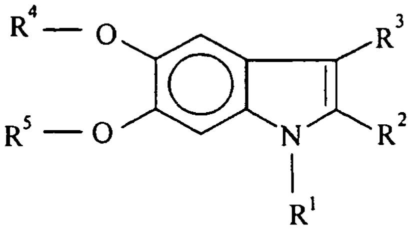

**(VIIb)**

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$ in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Wenn das erfindungsgemäße Mittel als farbgebende Komponente eine Kupplerkomponente enthält, werden als erfindungsgemäß bevorzugte Kupplerkomponenten verwendet:

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,

- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Bevorzugte direktziehende Farbstoffe, die in den erfindungsgemäßen Mitteln Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-vitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

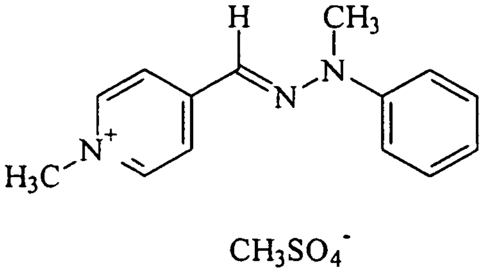

(DZ1)

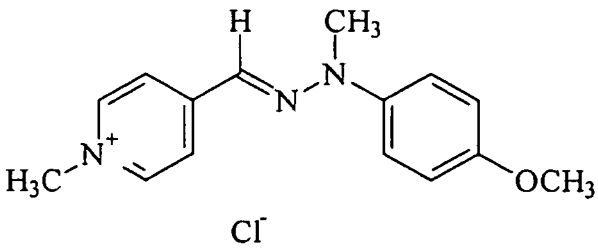

(DZ2)

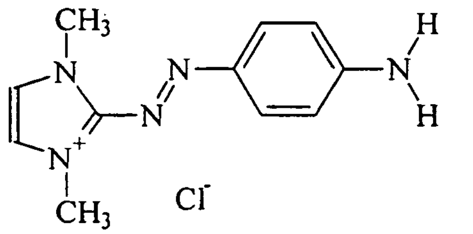

(DZ3)

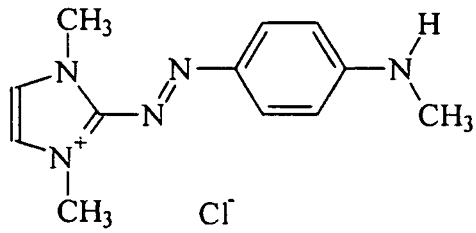

(DZ4)

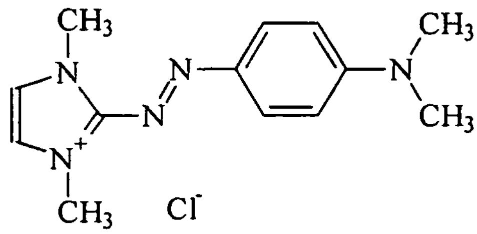

(DZ5)

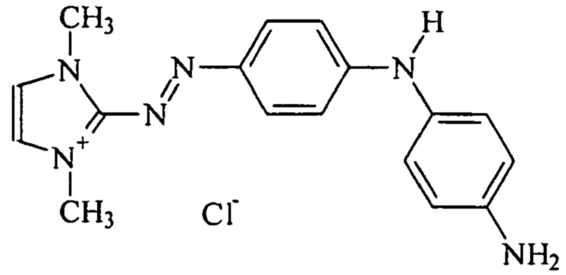

(DZ6)

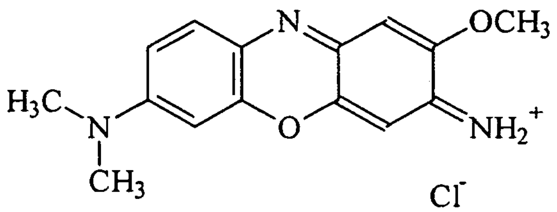

(DZ7)

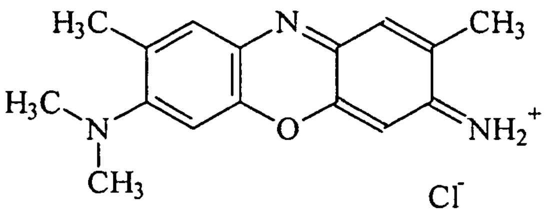

(DZ8)

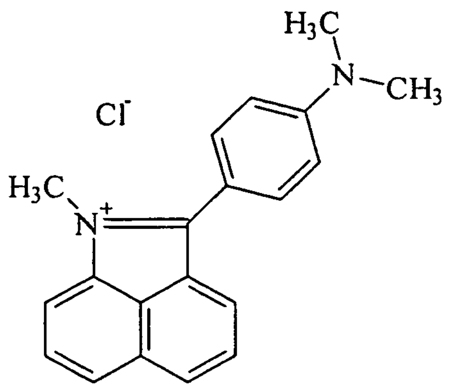

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Zusammensetzungen gemäß dieser Ausführungsform enthalten die direktziehenden Farbstoffe bevorzugt in einer Menge von 0,01 bis 20 Gew.-%, bezogen auf das gesamte Mittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Wenn das erfindungsgemäße Mittel Oxidationsfarbstoffvorprodukte enthält, kann die eigentliche oxidative Färbung der Fasern grundsätzlich mit Luftsauerstoff erfolgen. Bevorzugt wird jedoch ein chemisches Oxidationsmittel eingesetzt, besonders dann, wenn neben der Färbung mit den Oxidationsfarbstoffvorprodukten und/oder direktziehenden Farbstoffen ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie an Natriumborat in Frage. Soll das erfindungsgemäße Mittel als Kombinationspräparat in Form eines oxidativen Haarfärbe- und Konditioniermittels bereitgestellt werden, besteht es bevorzugt aus zwei Komponenten, nämlich einer Zubereitung, enthaltend die Farbstoffvorprodukte bzw. die direktziehenden Farbstoffe (die im weiteren als Farbstoffträger bezeichnet wird) und einer Oxidationsmittelzusammensetzung.

Ein zweiter Gegenstand der Erfindung ist daher ein Mittel, welches unmittelbar vor der Anwendung aus zwei Komponenten gemischt wird, wobei die erste Komponente als Farbstoffträger ein Mittel des ersten Erfindungsgegenstandes, enthaltend

- mindestens eine Entwicklerkomponente und gegebenenfalls mindestens eine Kupplerkomponente und gegebenenfalls
- mindestens einen direktziehenden Farbstoff

und die zweite Komponente eine Oxidationsmittelzusammensetzung, enthaltend mindestens ein chemisches Oxidationsmittel, insbesondere Wasserstoffperoxid, enthält.

Es ist erfindungsgemäß bevorzugt, die Verbindungen gemäß Formel (I) und das amphotere Polymer in den Farbstoffträger unter erhalt des zuvor beschriebenen erfindungsgemäßen Mittels einzuarbeiten. Es ist jedoch auch möglich, die Verbindung der Formel (I) beispielsweise in den Farbstoffträger und das amphotere Polymer in die Oxidationsmittelzusammensetzung zu formulieren, so dass bei Mischung der Komponenten das erfindungsgemäße Mittel erhalten wird.

Erfindungsgemäß kann das erfindungsgemäße Mittel auch zusammen mit einem Oxidationsaktivator auf das Haar aufgebracht werden, der die Oxidation der Farbstoffvorprodukte durch das Oxidationsmittel, aktiviert. Als Oxidationsmittel dient, wie zuvor erwähnt, Luftsauerstoff oder zusätzliche chemische Oxidationsmittel. Die Oxidationsaktivatoren werden bevorzugt ausgewählt aus der Gruppe, die gebildet wird aus Carbonaten, Hydrogencarbonaten, Carbamaten, Carbonsäureestern oder deren Salze, Aldehyden, insbesondere aliphatischen Aldehyden, 1,3-Dihydroxyaceton, Imidazol und seinen Derivaten, Alkali- und Ammoniumperoxidisulfaten, Metallionen, Iodiden, Chinonen und Enzymen.

Die Oxidationsaktivatoren sind bevorzugt in Mengen von 0.01 bis 5 Gel.%, bezogen auf das Gewicht des gesamten Mittels, in den erfindungsgemäßen Mitteln enthalten.

Geeignete Metallionen sind beispielsweise $Zn^{2+}$, $Cu^{2+}$, $Fe^{2}+$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,

- Uricase und Harnsäure oder deren Salze,
- Cholinoxidase und Cholin,
- Aminosäure-Oxidase und Aminosäuren.

Das eigentliche (oxidative) Haarfärbemittel wird zweckmäßigerweise unmittelbar vor der Anwendung durch Mischung der Oxidationsmittelzusammensetzung mit dem Farbstoffträger, bevorzugt im Gewichtsverhältnisbereich von 1 zu 4 bis 4 zu 1, insbesondere von 1 zu 2 bis 2 zu 1, hergestellt. Das dabei entstehende gebrauchsfertige Haarfärbepräparat sollte bevorzugt einen pH-Wert im Bereich von 6 bis 12, insbesondere von pH 7 bis 11, aufweisen. Besonders bevorzugt ist die Anwendung der Haarfärbemittel in einem schwach alkalischen Milieu.

Es wird angestrebt, daß der (insbesondere gelförmige und klare) Farbstoffträger fließfähig ist und bevorzugt eine Viskosität von höchstens 100 mPa·s (20 °C), bevorzugt von höchstens 20 mPa·s (20 °C) besitzt. Wird diese Zusammensetzung mit einer Oxidationsmittelzusammensetzung vermischt, weist der anwendungsfertige (insbesondere gelförmige und klare) Färbeansatz bevorzugt eine Viskosität von wenigstens 3 Pa·s (20 °C), bevorzugt von 8 bis 20 Pa·s (20 °C), auf (Viskositätsmessungen jeweils mit dem Rotationsviskosimeter Brookfield, Typ RTV, Spindel 4, 4 rpm).

Als erfindungsgemäß bevorzugte Oxidationsmittelzusammensetzung für die Ausführungsform als Färbemittel eignet sich hervorragend eine wässrige Zusammensetzung, enthaltend

| | |
|---|---|
| 3 bis 12 Gew.-% | Wasserstoffperoxid |
| 0,1 bis 5 Gew.-% | eines wasserlöslichen, synthetischen Tensids und |
| 1 bis 5 Gew.-% | eines dispergierten Acrylsäure- und/oder Methacrylsäure-Polymerisats oder -Copolymerisats. |

Sie kann aber auch ohne Wasserstoffperoxid oder im einfachsten Fall allein aus Wasser bestehen, so daß die Oxidation von Oxidationsfarbstoffvorprodukten durch den Luftsauerstoff herbeigeführt wird.

Als wasserlösliche synthetische Tenside können in der Oxidationsmittelzusammensetzung die zuvor für das erfindungsgemäße Mittel genannten optionalen Tenside aus anionischen, amphoteren, zwitterionischen und nichtionischen Tensiden oder Gemischen davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt:

Die Oxidationsmittelzusammensetzung enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit des alkalischen Farbstoffträgers, der Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich im wässrigen Medium zu lösen und die Viskosität der Lösung anzuheben.

Besonders günstig für den Viskositätsaufbau nach dem Vermischen ist der Gehalt an dispergierten Acrylsäure- und/oder Methacrylsäure-Polymerisat oder -Copolymerisat in der Oxidationsmittelzusammensetzung. Bevorzugte Dispersionen von entsprechenden Polymerisaten sind solche, welche die zuvor genannten bevorzugten Acrylsäure- und/oder Methycrylsäre-Polymerisate oder -Copolymerisate enthalten.

Die erfindungsgemäßen Mittel können zusätzlich alle auf diesen Gebieten bekannten und üblicherweise verwendeten Wirk- und Hilfsstoffe enthalten.

Ein dritter Gegenstand der vorliegenden Erfindung ist ein Verfahren zur Färbung und Konditionierung keratinischer Fasern, bei dem ein erfindungsgemäßes Mittel, enthaltend

- mindestens eine Entwicklerkomponente und gegebenenfalls mindestens eine Kupplerkomponente und gegebenenfalls
- mindestens einen direktziehenden Farbstoff

auf die Fasern aufgetragen wird und nach einer Einwirkzeit wieder abgespült wird.

Die Anwendungstemperaturen können in einem Bereich zwischen 15 und 40 **°C** liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein vierter Gegenstand ist eine Verpackungseinheit (Kit). welche mindestens ein getrennt konfektioniertes, erfindungsgemäßes Mittel des ersten Erfindungsgegenstandes enthält. Zusätzlich kann das Kit mindestens eine getrennt konfektionierte Oxidationsmittelzusammensetzung enthalten, insbesondere dann, wenn ein (oxidatives) Färbemittel bereitgestellt werden soll. Darüber hinaus kann das Kit zusätzlich optional eine Gebrauchsanweisung, Applikationshilfen,

eine zusätzliche Spülung (bevorzugt mit neutralem bis sauren pH-Wert) und/oder Schutzhandschuhe enthalten.

Eine Verpackungseinheit bildet sich erfindungsgemäß, wenn getrennt konfektionierte Zusammensetzungen durch eine gemeinsame Umverpackung, wie z.B. eine Faltschachtel oder eine Banderole, zusammengeführt werden. Im Sinne der Erfindung gilt es ebenso als Verpackungseinheit, wenn eine gemeinsame oder sequenzielle Benutzung getrennt konfektionierter Zusammensetzungen durch Anweisung in der Gebrauchsanleitung beziehungsweise in der Werbung vorgeschlagen wird.

**Beispiele**

1.0 Oxidatives Haarfärbemittel

Nachfolgende Farbstoffträger wurden nach einem bekannten Verfahren hergestellt.

| Rohstoff | Menge in Gew.-% | | | | | | | |
|---|---|---|---|---|---|---|---|---|
| | E1 | V1 | E2 | E3 | V2 | V3 | V4 | V5 |
| Dehydol® LS2 [1] | 10,00 | 10,00 | 10,00 | 10,00 | 10,00 | 10,00 | 10,00 | 10,00 |
| Kokoslorol® [2] | 8,00 | 8,00 | 8,00 | 8,00 | 8,00 | 8,00 | 8,00 | 8,00 |
| Edenor® PK1805 [3] | 6,75 | 6,75 | 6,75 | 6,75 | 6,75 | 6,75 | 6,75 | 6,75 |
| Isopropanol | 14,50 | 14,50 | 14,50 | 14,50 | 14,50 | 14,50 | 14,50 | 14,50 |
| 1,2-Propylenglykol | 7,85 | 7,85 | 6,75 | 6,75 | 6,75 | 6,75 | 6,75 | 6,75 |
| Texapon® NSO UP [4] | 3,50 | 3,50 | 3,50 | 3,50 | 3,50 | 3,50 | 3,50 | 3,50 |
| Monoethanolamin | 5,50 | 5,50 | 8,00 | 8,00 | 8,00 | 8,00 | 8,00 | 8,00 |
| L-Arginin | 1,00 | 1,00 | 1,00 | 1,00 | 1,00 | 1,00 | 1,00 | 1,00 |
| Natriumsulfit | 0,40 | 0,40 | 0,20 | 0,20 | 0,20 | 0,20 | 0,20 | 0,20 |
| Gluadin® W 40 [5] | 3,00 | 3,00 | 3,00 | 3,00 | 3,00 | 3,00 | 3,00 | 3,00 |
| Turpinal® SL [6] | 0,20 | 0,20 | 0,20 | 0,20 | 0,20 | 0,20 | 0,20 | 0,20 |
| Parfüm | 0,60 | 0,60 | 0,60 | 0,60 | 0,60 | 0,60 | 0,60 | 0,60 |
| Merquat® 280 [7] | 3,00 | - | 3,00 | 3,00 | 3,00 | 3,00 | 3,00 | - |
| Miranol® C2M SF [8] | 2,00 | - | 2,00 | 2,00 | - | - | - | 2,00 |
| Dehyton® K[9] | - | - | - | - | 2,00 | - | - | - |
| Dehyton® PS [10] | - | - | - | - | - | - | 2,00 | - |
| Lowenol® S [11] | - | - | - | 2,00 | - | - | - | - |
| p-Toluylendiaminsulfat | 0,21 | 0,21 | 0,05 | 0,05 | 0,05 | 0,05 | 0,05 | 0,05 |
| 3-Methyl-p-aminophenol | 0,50 | 0,50 | - | - | - | - | - | - |
| Resorcin | 0,28 | 0,28 | 0,01 | 0,01 | 0,01 | 0,01 | 0,01 | 0,01 |
| 4-Clorresorcin | - | - | 0,01 | 0,01 | 0,01 | 0,01 | 0,01 | 0,01 |
| 5-Amino-2-methylphenol | 0,36 | 0,36 | - | - | - | - | - | - |
| m-Aminophenol | - | - | 0,001 | 0,001 | 0,001 | 0,001 | 0,001 | 0,001 |
| 1,2,3,4-Tetrahydro-6-nitrochinoxalin | 0,10 | 0,10 | - | - | - | - | - | - |

(fortgesetzt)

| Rohstoff | Menge in Gew.-% | | | | | | | |
|---|---|---|---|---|---|---|---|---|
| | E1 | V1 | E2 | E3 | V2 | V3 | V4 | V5 |
| Wasser | <----------------------------------------ad 100------------------------------------------> | | | | | | | |

1 $C_{12}$-$C_{14}$ Fettalkohol mit 2 Einheiten Ethylenoxid (INCI-Bezeichnung: Laureth-2) (Hersteller: COGNIS)
2 $C_{12}$-$C_{18}$-Fettalkohol (INCI-Bezeichnung: Coconut Alcohol) (Hersteller: COGNIS)
3 Kaliumoleat (Hersteller: COGNIS)
4 Natriumlaurylethersulfat (27 % Aktivsubstanz; INCI: Sodium Laureth Sulfate) (Hersteller : COGNIS)
5 Weizenproteinhydrolysat (Aktivsubstanz 40 Gew.-%) (INCI-Bezeichnung: Aqua (Water), Hydrolyzed Wheat Protein, Sodium Benzoate, Phenoxyethanol, Methylparaben, Propylparaben) (Hersteller: Gelita)
6 1-Hydroxyethan-1,1-diphosphonsäure (INCI-Bezeichnung: Etidronic Acid, Aqua (Water)) (Hersteller: Solutia)
7 Dimethyldiallylammoniumchlorid Acrylsäure Copolymer (35 % Aktivsubstanz; INCI-Bezeichnung: Polyquaternium-22) (Hersteller: Nalco)
8 INCI-Bezeichnung: Disodium Cocoamphodipropionate (Hersteller: RHODIA)
9 N,N-Dimethyl-N-($C_8$-$C_{18}$-kokosamidopropyl)ammoniumacetobetain (ca. 30 % Aktivsubstanz; INCI-Bezeichnung: Aqua (Water), Cocamidopropyl Betaine) (Hersteller: COGNIS)
10 N-(Kokosfettsäureamidoethyl)-N-(2-hydroxyethyl)glycin Natrium Salz (37,5 % Aktivsubstanz; INCI-Bezeichnung: Disodium Cocoamphodiacetate)
11 Bis(2-hydroxyethyl)sojaalkylamindioleat (97 Gew.-% Aktivsubstanz, INCI-Bezeichnung: Dihydroxyethyl Soyamine Dioleate) (Hersteller: Lowenstein)

Unmittelbar vor der Anwendung am Haar wurde je ein Färbeträger mit nachfolgender Oxidationsmittelzubereitung zu einem anwendungsbereiten Färbemittel im Gewichtsverhältnis von 5 zu 4 vermischt:

| | Gew.-% |
|---|---|
| Dipicolinsäure | 0,62 |
| Natriumpyrophosphat | 0,03 |
| Turpinal® SL | 1,50 |
| Texapon® NSO UP | 2,00 |
| Aculyn® 33 A [12] | 12,00 |
| Wasserstoffperoxid (50 %ige wässrige Lösung) | 12,00 |
| Wasser | ad 100 |

12 Acrylsäure Copolymer (30% Aktivsubstanz, INCI-Bezeichung: Acrylates Copolymer) (Hersteller: Rohm & Haas)

Die Ausfärbung erfolgte stets auf Haarsträhnen (Kerling Euronaturhaar Code 6/0, ungeschädigt). Die Haarsträhnen wurden im Rahmen einer Vorbehandlung einmal mit dem Handelsprodukt Poly Blond® (Schwarzkopf & Henkel) blondiert und 24 Stunden gelagert.

1.1 Quantitative Bestimmung der Reduktion der Nasskämmbarkeit

Als Referenzmessung wurde vor der Anwendung der Färbemittel die Kämmkraft jeder Haarsträhne bestimmt (siehe Punkt 1.2).

Je Farbstoffträger E1 und V1 wurden 20 Haarsträhnen angefeuchtet und mit dem Handtuch getrocknet, so dass eine Restfeuchtigkeit im Haar verblieb. Danach wurde je Strähne ein anwendungsbereites klares, gelförmiges Färbemittel in einem Flottenverhältnis von 4 g Anwendungsmischung pro g Haar aufgetragen und über einen Zeitraum von 30 Minuten einwirken gelassen. Anschließend wurden die Strähnen gründlich mit Leitungswasser gespült.

Abschließend wurde erneut die Kämmkraft jeder Haarsträhne bestimmt (siehe Punkt 1.2).

Ergebnis:

Das erfindungsgemäße Mittel mit dem Farbstoffträger E1 bewirkte eine Reduktion der Nasskämmbarkeitskraft um 35%.

Das nicht erfindungsgemäße Mittel mit dem Farbstoffträger V1 bewirkte eine Erhöhung der Nasskämmbarkeitskraft um 15%.

1.2 Durchführung der Kämmkraftmessung

Vor der Kraftmessung wurden die Einzelsträhnen ca. 1 Minute in einer Vorkämmapparatur nass gekämmt. Unmittelbar im Anschluss daran wurde von jeder einzelnen Strähne die Arbeit ermittelt, die für 10 Kämmungen aufgewendet werden musste. Die Messergebnisse der 20 Strähnen wurden gemittelt.

Die so erhaltenen gemittelten Messwerte für die Kämmkraft vor (Referenz) und nach der Färbung wurden verglichen und die Änderung der Kämmkraft auf die Referenzmessung bezogen. Die Änderung wurde wie folgt berechnet:

$$\text{Änderung} = (1 - [\text{Kämmarbeit nachher}])/[\text{Kämmarbeit Referenz}]) \cdot 100$$

1.3 Beurteilung der Trübung und der Avivage

Die Farbstoffträger-Rezepturen E1, E2, E3, V1 und V5 sowie die entsprechend resultierenden Anwendungsmischungen lieferten ein klares Gel. Alle anderen Mittel wiesen eine erhebliche Trübung auf.

Je Strähne wurde ein anwendungsbereites klares, gelförmiges Färbemittel in einem Flottenverhältnis von 4 g Anwendungsmischung pro g Haar aufgetragen und über einen Zeitraum von 30 Minuten einwirken gelassen. Anschließend wurden die Strähnen gründlich mit Leitungswasser gespült und mit dem Handtuch getrocknet.

Die Avivage der Rezepturen E2, E3, V2 bis V5 wurde von 6 fachkundigen Personen anhand der Nasskämmbarkeit bewertet. Dabei wurde der Bewertung ein Notensystem von 1 bis 5 (1 = sehr gut, 5 = sehr schlecht) zugrunde gelegt. Ergebnisse mit Noten von 1 bis 2- erfüllen die Anforderungen an ein hervorragendes Konditioniermittel zur Lösung der erfindungsgemäßen Ausgabe.

Die Ergebnisse waren wie folgt:

| Rezeptur | Avivage | Trübung |
|---|---|---|
| E2 | 2 | nein |
| E3 | 2- | nein |
| V2 | 3 | ja |
| V3 | 3 | ja |
| V4 | 3 | ja |
| V5 | 3- | nein |

Nur die erfindungsgemäßen Mittel, welche die Verbindung gemäß Formel (I) in Kombination mit dem amphoteren Polymer Polyquaternium-22 enthalten, besitzen eine gesteigerte Avivageleistung. Sowohl das amphotere Polymer Polyquaternium-22 allein (V3), als auch die Verbindung gemäß Formel (I) allein (V5) ergeben eine unzureichende Avivage.

Die Kombination des amphoteren Polymers Polyquaternium-22 mit anderen amphoteren Tensiden ohne die Verbindung gemäß Formel (I) (V2 und V4) ergeben ebenso eine schlechte Avivage und darüber hinaus jeweils eine Trübung.

## Patentansprüche

1. Wasserhaltiges Mittel zur Konditionierung keratinhaltiger Fasern, insbesondere menschlicher Haare, **dadurch gekennzeichnet, daß** es

   - mindestens ein amphoteres Tensid gemäß Formel **(I)** und/oder dessen physiologisch verträgliches Salz,

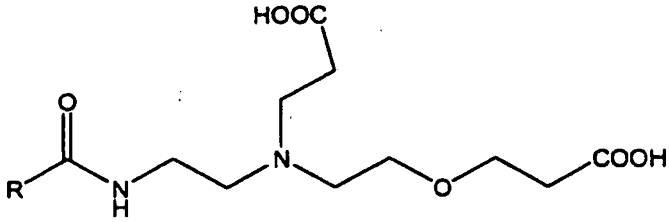

(I)

worin R eine lineare oder verzweigte, gesättigte oder ungesättigte ($C_{10}$ - $C_{22}$)-Alkylgruppe bedeutet,

- mindestens ein amphoteres Polymer
und
- als Oxidationsfarbsoffvorprodukt mindestens eine Entwicklerkomponente und gegebenenfalls mindestens eine Kupplerkomponente enthält.

**2.** Mittel nach Anspruch 1 **dadurch gekennzeichnet, daß** die Verbindungen der Formel (I) in einer Menge von 0,05 bis 30 Gew.-%, bezogen auf das Gewicht des Mittels, enthalten sind.

**3.** Mittel nach einem der Ansprüche 1 oder 2, **dadurch gekennzeichnet, dass** die amphoteren Polymere zwitterionische Polymere sind.

**4.** Mittel nach einem der Ansprüche 1 bis 3, **dadurch gekennzeichnet, daß** eine kationische Gruppe der amphoteren Polymere sich ableitet von mindestens einem Monomer, ausgewählt aus

(M1) Monomeren mit quartären Ammoniumgruppen der allgemeinen Formel (II),

$$R^1\text{-}CH{=}CR^2\text{-}CO\text{-}Z\text{-}(C_nH_{2n})\text{-}N^{(+)}R^3R^4R^5\ A^{(-)} \qquad (II)$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,
(M2) Monomeren mit quartären Ammoniumgruppen der allgemeinen Formel (III),

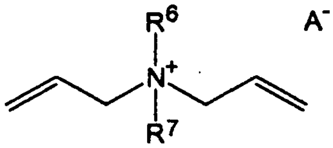

(III)

worin $R^6$ und $R^7$ unabhängig voneinander stehen für eine ($C_1$ bis $C_4$)-Alkylgruppe, insbesondere für eine Methylgruppe und
$A^-$ das Anion einer organischen oder anorganischen Säure ist.

**5.** Mittel nach einem der Ansprüche 1 bis 4, **dadurch gekennzeichnet, dass** sich eine anionische Gruppe des amphoteren Polymers ableitet von einem Monomer der Formel (IV) oder deren Salze mit einer organischen oder anorganischen Säure,

$$R^8\text{-}CH{=}CR^8\text{-}COOH \qquad (IV)$$

in denen $R^3$ und $R^9$ unabhängig voneinander Wasserstoff oder Methylgruppen sind.

**6.** Mittel nach einem der Ansprüche 1 bis 5, **dadurch gekennzeichnet, daß** das amphotere Polymer ausgewählt wird aus Copolymeren des Monomers (M2) und des Monomers gemäß Formel (IV).

**7.** Mittel nach einem der Ansprüche 1 bis 6, **dadurch gekennzeichnet, dass** die amphoteren Polymere in einer Menge von 0,01 bis 10 Gew.-% bezogen auf das Gewicht des gesamten Mittels, enthalten sind.

**8.** Mittel nach einem der Ansprüche 1 bis 7, **dadurch gekennzeichnet, daß** es in Form eines klaren Gels vorliegt.

**9.** Mittel nach einem der Ansprüche 1 bis 8, **dadurch gekennzeichnet, dass** es zusätzlich eine Kombination aus

a) 0,5 bis 10 Gew.-% eines Anlagerungsproduktes von 1 bis 5 Mol Ethylenoxid an einen linearen Fettalkohol mit 8 bis 22 C-Atomen,
b) 6,0 bis 15 Gel.-% eines gesättigten oder ungesättigten, linearen oder verzweigten Alkohols mit 8 bis 36 C-Atomen und
c) 0,1 bis 15 Gew.-% einer flüssigen Fettsäure mit 16 bis 22 C-Atomen in Form einer wasserlöslichen Seife enthält.

**10.** Mittel nach einem der Ansprüche 1 bis 9, **dadurch gekennzeichnet, dass** es zusätzlich 0,5 bis 10 Gew.-% eines Anlagerungsproduktes von 15 bis 100 Einheiten Ethylenoxid pro Molekül eines linearen Fettalkohols mit 8 bis 22 C-Atomen, enthält.

**11.** Mittel nach einem der Ansprüche 1 bis 10, **dadurch gekennzeichnet, dass** es zusätzlich 3 bis 70 Gel.-% mindestens eines niedermolekularen Monoalkohols. und/oder mindestens eines Polyols enthält.

**12.** Mittel nach einem der Ansprüche 1 bis 11, **dadurch gekennzeichnet, daß** es zusätzlich mindestens einen direktziehenden Farbstoff enthält.

**13.** Mittel, welches unmittelbar vor der Anwendung aus zwei Komponenten gemischt wird, **dadurch gekennzeichnet, daß** die erste Komponente ein Mittel gemäß einem der Ansprüche 1 bis 12 ist und die zweite Komponente eine Oxidationsmittelzusammensetzung, enthaltend mindestens ein chemisches Oxidationsmittel, insbesondere Wasserstoffperoxid, ist.

**14.** Verfahren zur Konditionierung keratinhaltiger Fasern, bei dem ein Mittel nach einem der Ansprüche 1 bis 13 auf die Fasern aufgetragen wird und nach einer Einwirkzeit wieder abgespült wird.

**15.** Kit, enthaltend mindestens ein getrennt konfektioniertes Mittel gemäß einem der Ansprüche 1 bis 12 in Kombination mit mindestens einer getrennt konfektionierten Oxidationsmittelzusammensetzung.

## Claims

**1.** A water-containing agent for the conditioning of keratinic fibers, in particular human hair, **characterized in that** it contains

- at least one amphoteric surfactant according to formula (I) and/or its physiologically acceptable salt,

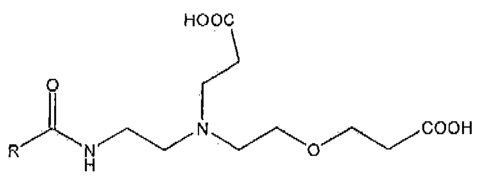

(I)

wherein R stands for a linear or branched, saturated or unsaturated $C_{10}$-$C_{22}$ alkyl group,

- at least one amphoteric polymer and
- as a oxidation dye precursor, at least one developer component and if necessary at least one coupler component.

**2.** The agent according to claim 1, **characterized in that** the compounds of formula (I) are contained in an amount from 0.05 to 30% by weight, based on the weight of the agent.

**3.** The agent according to any of claims 1 or 2, **characterized in that** the amphoteric polymers are zwitterionic polymers.

**4.** The agent according to any of claims 1 to 3, **characterized in that** a cationic group of the amphoteric polymers is derived from at least one monomer selected from

(M1) monomers with quaternary ammonium groups of general formula (II),

$$R^1\text{-}CH{=}CR^2\text{-}CO^2\text{-}(C_nH_{2n})\text{-}N^{(+)}R^3R^4R^5\ A^{(-)} \qquad (II)$$

wherein $R^1$ and $R^2$, independently of each other, stand for a hydrogen or a methyl group and $R^3$, $R^4$ and $R^5$, independently of each other, stand for alkyl groups with 1-4 carbon atoms, Z stands for an NH group or an oxygen atom, n is an integer from 2 to 5 and $A^{(-)}$ is the anion of an organic or inorganic acid,
(M2) monomers with quaternary ammonium groups of general formula (III),

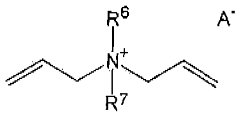

(III)

wherein $R^6$ and $R^7$ independently of each other stand for a $C_1$-$C_4$ alkyl group, in particular for a methyl group and $A^-$ is the anion of an organic or inorganic acid.

**5.** The agent according to any of claims 1 to 4, **characterized in that** an anionic group of the amphoteric polymer is derived from a monomer of formula (IV) or their salts with an organic or inorganic acid,

$$R^8\text{-}CH{=}CR^8\text{-}COOH \qquad (IV)$$

wherein $R^8$ and $R^9$ independently of each other are hydrogen or methyl groups.

**6.** The agent according to any of claims 1 to 5, **characterized in that** the amphoteric polymer is selected from copolymers of the monomer (M2) and of the monomer according to formula (IV).

**7.** The agent according to any of claims 1 to 6, **characterized in that** the amphoteric polymers are contained in an amount from 0.01 to 10% by weight, based on the weight of the total agent.

**8.** The agent according to any of claims 1 to 7, **characterized in that** it is present as a transparent gel.

**9.** The agent according to any of claims 1 to 8, **characterized in that** it further contains a combination of

a) 0.5 to 10% by weight of an addition product of 1 to 5 moles of ethylene oxide on a linear fatty alcohol with 8 to 22 C atoms,
b) 6.0 to 15% by weight of a saturated or unsaturated, linear or branched alcohol with 8 to 36 C atoms, and
c) 0.1 to 15% by weight of a liquid fatty acid with 16 to 22 C atoms as a water-soluble soap.

**10.** The agent according to any of claims 1 to 9, **characterized in that** it further contains 0.5 to 10% by weight of an addition product of 15 to 100 ethylene oxide units per molecule of a linear fatty alcohol with 8 to 22 C atoms.

**11.** The agent according to any of claims 1 to 10 **characterized in that** it further contains 3 to 70% by weight of at least one low molecular weight monoalcohol and/or at least one polyol.

**12.** The agent according to any of claims 1 to 11, **characterized in that** it further contains at least one substantive dye.

**13.** The agent, which is directly mixed out of two components before the application, **characterized in that** the first component is an agent according to any of claims 1 to 12 and the second component is an oxidation agent composition

containing at least one chemical oxidation agent, in particular hydrogen peroxide.

**14.** A method for conditioning and oxidative dyeing of keratinic fibers, wherein an agent according to any of claims 1 to 13 is applied on the fibers and is again rinsed after a duration of action.

**15.** A kit containing at least one separately ready-made agent according to any of claims 1 to 12 in combination with at least one separately ready-made oxidation agent composition.

## Revendications

**1.** Agent aqueux pour la revitalisation capillaire de fibres kératiniques, en particulier de cheveux humains, **caractérisé en ce qu'**il contient

- au moins un agent tensioactif amphotère répondant à la formule (I) et/ou son sel physiologiquement acceptable

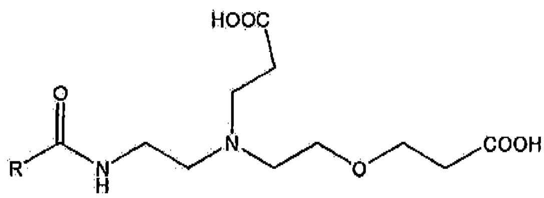

(I)

dans laquelle R représente un groupe alkyle en $C_{10}$-$C_{22}$ linéaire ou ramifié, saturé ou insaturé ;
- au moins un polymère amphotère ; et
- à titre de précurseur de colorant d'oxydation, au moins un composant faisant office de développeur et le cas échéant au moins un composant faisant office de coupleur.

**2.** Agent selon la revendication 1, **caractérisé en ce que** les composés répondant à la formule (I) sont contenus en une quantité de 0,05 à 30 % en poids, rapportés au poids de l'agent.

**3.** Agent selon la revendication 1 ou 2, **caractérisé en ce que** les polymères amphotères sont des polymères zwitterioniques.

**4.** Agent selon l'une quelconque des revendications 1 à 3, **caractérisé en ce qu'**un groupe cationique des polymères amphotères dérive d'au moins un monomère choisi parmi

(M1) des monomères contenant des groupes d'ammonium quaternaires répondant à la formule générale (II) :

$$R^1\text{-}CH{=}CR^2\text{-}CO\text{-}Z\text{-}(C_nH_{2n})\text{-}N^{(+)}R^3R^4R^5\ A^{(-)} \qquad (II)$$

dans laquelle $R^1$ et $R^2$ représentent, indépendamment l'un de l'autre, un atome d'hydrogène ou un groupe méthyle et $R^3$, $R^4$ et $R^5$ représentent, indépendamment l'un de l'autre, des groupes alkyle contenant de 1 à 4 atomes de carbone, Z représente un groupe NH ou un atome d'oxygène, n représente un nombre entier de 2 à 5 et $A^{(-)}$ représente l'anion d'un acide organique ou inorganique ;
(M2) des monomères contenant des groupes d'ammonium quaternaires répondant à la formule générale (III) :

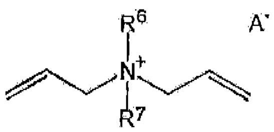

(III)

dans laquelle $R^6$ et $R^7$ représentent, indépendamment l'un de l'autre, un groupe alkyle en $C_1$-$C_4$, en particulier un groupe méthyle, et $A^-$ représente l'anion d'un acide organique ou inorganique.

**5.** Agent selon l'une quelconque des revendications 1 à 4, **caractérisé en ce qu'**un groupe anionique du polymère amphotère dérive d'un monomère répondant à la formule (IV) ou de ses sels avec un acide organique ou inorganique

$$R^8\text{-}CH{=}CR^5COOH \qquad (IV)$$

dans laquelle $R^8$ et $R^9$ représentent, indépendamment l'un de l'autre, un atome d'hydrogène ou des groupes méthyle.

**6.** Agent selon l'une quelconque des revendications 1 à 5, **caractérisé en ce que** le polymère amphotère est choisi parmi des copolymères du monomère (M2) et du monomère répondant à la formule (IV).

**7.** Agent selon l'une quelconque des revendications 1 à 6, **caractérisé en ce que** les polymères amphotères sont contenus en une quantité de 0,01 à 10 % en poids, rapportés au poids de la totalité de l'agent.

**8.** Agent selon l'une quelconque des revendications 1 à 7, **caractérisé en ce qu'**il est présent sous la forme d'un gel clair.

**9.** Agent selon l'une quelconque des revendications 1 à 8, **caractérisé en ce qu'**il contient en outre une combinaison de :

a) à concurrence de 0,5 à 10 % en poids, un produit de fixation par addition de 1 à 5 mol d'oxyde d'éthylène à un alcool gras linéaire contenant de 8 à 22 atomes de carbone ;
b) à concurrence de 6,0 à 15 % en poids, un alcool linéaire ou ramifié, saturé ou insaturé, contenant de 8 à 36 atomes de carbone ; et
c) à concurrence de 0,1 à 15 % en poids, un acide gras liquide contenant de 16 à 22 atomes de carbone sous la forme d'un savon soluble dans l'eau.

**10.** Agent selon l'une quelconque des revendications 1 à 9, **caractérisé en ce qu'**il contient en outre, à concurrence de 0,5 à 10 % en poids, un produit de fixation par addition de 15 à 100 unités d'oxyde d'éthylène par molécule d'un alcool gras linéaire contenant de 8 à 22 atomes de carbone.

**11.** Agent selon l'une quelconque des revendications 1 à 10, **caractérisé en ce qu'**il contient en outre, à concurrence de 3 à 70 % en poids, au moins un alcool monovalent à bas poids moléculaire et/ou au moins un polyol.

**12.** Agent selon l'une quelconque des revendications 1 à 11, **caractérisé en ce qu'**il contient en outre au moins un colorant montant directement sur la fibre.

**13.** Agent, qui est mélangé directement avant son utilisation, à partir de deux composants, **caractérisé en ce que** le premier composant représente un agent selon l'une quelconque des revendications 1 à 12 et le deuxième composant représente une composition d'agent d'oxydation contenant au moins un agent d'oxydation chimique, en particulier du peroxyde d'hydrogène.

**14.** Procédé pour la revitalisation capillaire et la teinture par oxydation de fibres kératiniques, dans lequel on applique sur les fibres un agent selon l'une quelconque des revendications 1 à 13 et on l'élimine par rinçage après l'avoir laissé agir pendant un certain temps.

**15.** Nécessaire contenant au moins un agent confectionné de manière séparée, selon l'une quelconque des revendications 1 à 12, en combinaison avec au moins une composition d'agent d'oxydation confectionnée de manière séparée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4507280 A
- US 62211389 B
- EP 640335 B1
- GB 2104091 A
- EP 47714 A
- EP 217274 A
- EP 283817 A
- DE 2817369
- DE 3929973
- US 3101300 A
- US 3101301 A
- DE 3843713 A1
- DE 3504242
- GB 870994 A
- DE 1164095
- DE 3445549
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- EP 998908 A2


### In der Beschreibung aufgeführte Nicht-Patentliteratur


- **P.J. FLORY.** Faraday discussions of the chemical society. *Gels and Gelling Processes,* 1974, 7-18
- **HAPPI.** *Household Pers. Prod. Ind.,* 1993, vol. 30 (2), 58-64
- *Angewandte Chemie,* 1952, vol. 64, 213-20
- **KH. SCHRADER.** Grundlagen und Rezepturen der Kosmetika. Hüthig Buch Verlag, 1989